(19) Europäisches Patentamt
European Patent Office
Office européen des brevets

(11) Publication number: **0 080 305**
**A1**

# (12) EUROPEAN PATENT APPLICATION

(21) Application number: 82306028.0

(22) Date of filing: 11.11.82

(51) Int. Cl.³: C 07 H 19/08, A 61 K 31/70

(30) Priority: 19.11.81 GB 8134827
11.03.82 GB 8207104
23.09.82 GB 8227198

(43) Date of publication of application: 01.06.83
Bulletin 83/22

(84) Designated Contracting States: BE CH DE FR GB IT LI NL SE

(71) Applicant: **BEECHAM GROUP PLC, Beecham House Great West Road, Brentford Middlesex (GB)**

(72) Inventor: **Harnden, Michael Raymond, 47 Guildford Road, Horsham Sussex (GB)**
Inventor: **Bailey, Stuart, 14 Overdale, Dorking Surrey (GB)**

(74) Representative: **Russell, Brian John et al, European Patent Attorney Beecham Pharmaceuticals Great Burgh Yew Tree Bottom Road, Epsom Surrey, KT18 5XQ (GB)**

(54) **Antiviral 2'-deoxyuridines, their preparation and use.**

(57) Compounds of formula (I):

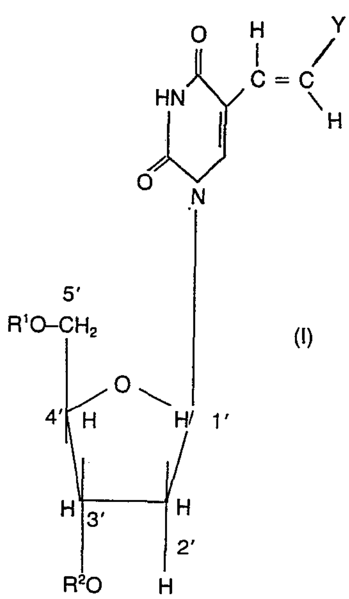

(I)

or a salt thereof, in which Y is a halogen atom, and each of $R^1$ and $R^2$ is a hydrogen atom or an acylamino group of the formula (IA):

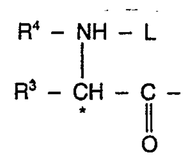

(IA)

which group is optionally N-alkylated or acylated, wherein $R^3$ is a hydrogen atom, or a $C_{1-6}$ alkyl, phenyl, optionally substituted $C_{1-6}$ phenylalkyl, $C_{1-6}$ hydroxyalkyl, $C_{1-6}$ aminoalkyl, optionally N-substituted with $C_{1-6}$ alkoxycarbonyl, $C_{1-6}$ thioalkyl, $C_{1-6}$ alkylthioalkyl, $C_{1-6}$ carboxyalkyl or $C_{1-6}$ carboxamidoalkyl group, $R^4$ is a hydrogen atom or together with $R^3$ forms the remaining members of a heterocyclic ring, and L is a hydrogen atom or a protecting group, provided that $R^1$ and $R^2$ are not simultaneously hydrogen atoms, are useful in the treatment of viral infections, particularly those caused by herpes viruses.

EP 0 080 305 A1

TITLE MODIFIED
see front page

## ANTIVIRAL AGENTS, THEIR PREPARATION AND USE

This invention relates to certain deoxyuridine compounds which have antiviral activity.

U.K. Patent Specification No. 1,601,020 discloses 5-(2-halogenovinyl)-2'-deoxyuridines which have antiviral activity selective against herpes virus.

We have now found a group of mono- and di-aminoacyl derivatives of 5-(2-halogenovinyl)-2'-deoxyuridine which have antiviral activity, and which are useful in the treatment of infections caused by herpes viruses, such as herpes simplex type 1, herpes simplex type 2 and varicella.

According to the present invention there is provided an ester of the formula (I):

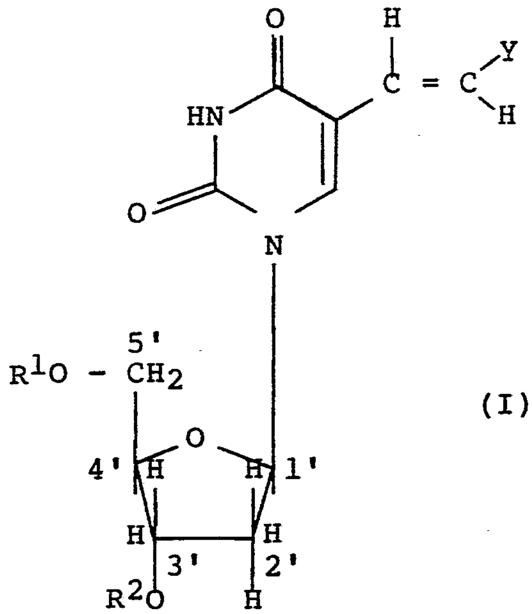

(I)

or a salt thereof,
in which Y is a halogen atom, and each of $R^1$ and $R^2$ is a hydrogen atom or an acylamino group of the formula (IA):

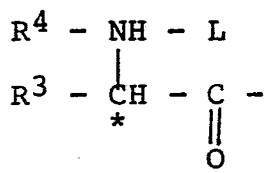

(IA)

which group is optionally N-alkylated or acylated, wherein $R^3$ is a hydrogen atom, or a $C_{1-6}$ alkyl, phenyl, optionally substituted $C_{1-6}$ phenylalkyl, $C_{1-6}$ hydroxyalkyl, $C_{1-6}$ aminoalkyl, optionally N-substituted with $C_{1-6}$ alkoxycarbonyl, $C_{1-6}$ thioalkyl, $C_{1-6}$ alkylthioalkyl, $C_{1-6}$ carboxyalkyl or $C_{1-6}$ carboxamido-alkyl group, $R^4$ is a hydrogen atom or together with $R^3$ forms the remaining members of a heterocyclic ring, and L is a hydrogen atom or a protecting group, provided that $R^1$ and $R^2$ are not simultaneously hydrogen atoms.

Preferably, Y is a bromine atom.

A preferred value for L is the tertiary butoxycarbonyl group.

Suitably, $R^3$ is an optionally substituted phenylalkyl group, preferably a benzyl group, optionally substituted by halogen, preferably fluorine.

When $R^3$ is a $C_{1-6}$ alkyl group, it is preferably methyl, iso-propyl or iso-butyl.

When $R^3$ and $R^4$ complete a heterocyclic ring, the ring is preferably a 5 to 7 membered saturated ring with no additional hetero-atom present therein.

Salts of the compounds of formula (I) are suitably acid addition salts formed with the amino group on the acylamino group of formula (IA). Examples of these salts are pharmaceutically acceptable inorganic salts such as the sulphate, nitrate, phosphate, borate, hydrochloride and hydrobromide, and pharmaceutically acceptable organic acid addition salts such as the acetate and trifluoroacetate.

The group of formula (IA) has an asymmetric carbon atom, marked with an asterisk in the formula. The compounds of formula (I) therefore, exist in two stereoisomeric forms, and the present invention encompasses both stereoisomers alone or admixed in any proportion.

The compounds of formula (I) may be prepared by reacting a compound of formula (II):

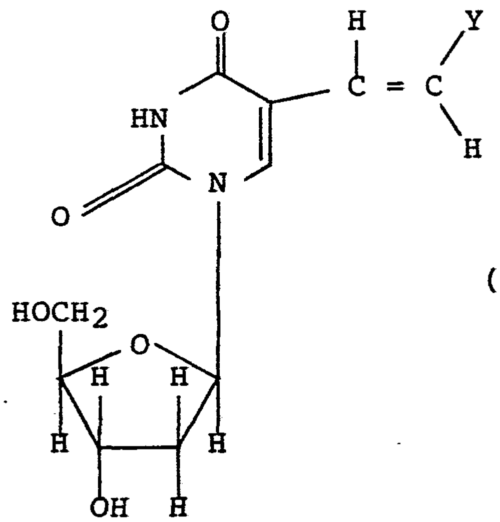

(II)

in which Y is as defined in formula (I), with an N-protected 2-amino carboxylic acid active ester, of formula (III):

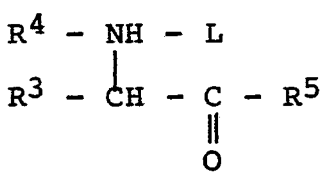

(III)

in which $R^3$ and $R^4$ are as defined in formula (I), L is a protecting group, and $R^5$ is an active ester group, and optionally thereafter removing the protecting group L. $R^5$ may be any suitable active ester group, but preferred groups are the N-hydroxysuccinimide group and the p-nitrophenyl group.

The reaction is preferably carried out in an inert solvent, such as dry tetrahydrofuran. The deprotection of the compounds of formula (I) is suitably carried out by treatment of the compounds with an acid, preferably trifluoroacetic or sulphuric acid.

Preferred salts of the compounds of formula (I) are those which are formed as a result of the acid deprotection reaction. For example, treatment with sulphuric acid or trifluoroacetic acid provides the bisulphate or trifluoroacetate salts respectively.

The reaction between the compounds of formulae (II) and (III) produces a mixture of three compounds, wherein:

(a) $R^1$ = hydrogen and $R^2$ = 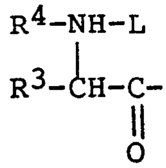

(b) $R^1$ = 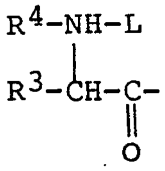 and $R^2$ = hydrogen

(c) $R^1$ and $R^2$ are both 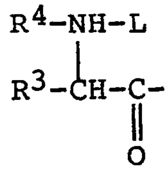

The three compounds may be separated chromatographically, suitably by column chromatography on silica gel.

The compounds of formula (I) may be formulated for use in a pharmaceutical composition. Accordingly, in a further aspect of the invention, there is provided a pharmaceutical composition which comprises a compound of the formula (I) together with a pharmaceutically acceptable carrier or excipient.

Compositions which may be given by the oral route may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol,

glycerine, saline and water to which flavouring or colouring agents may be added to form syrups. The compounds may also be presented with a sterile liquid carrier for injection.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books, and the British Pharmacopaeia.

The composition for application to the eyes may be a conventional eye-drop composition well known in the art.

Preferably, the compositions of this invention are in unit dosage form or in some other form that the patient may administer to himself a single dose. A suitable dosage unit might contain from 50 mg to 1g of active ingredient, for example 100 mg to 500 mg. Such doses may be administered 1 to 4 times a day or more usually 2 or 3 times a day. The effective dose of compound depends on the particular compound employed, but is in general in the range of from 1.0 mg/kg/day to 20 mg/kg of body weight per day or more usually 2.0 mg/kg/day to 10 mg/kg/day.

In a further aspect of the invention there is provided a method of treating viral infections in human or non-human animals, which comprises administering to the sufferer an effective amount of a compound of formula (I) or a salt thereof.

The following Examples illustrate the invention.

Examples 1, 2 and 3

Preparation of E-5-(2-bromovinyl)-2'-deoxyuridine-3'(5')-0- and 3',5'-di-0-(tert.butoxycarbonylphenylalanyl) esters

A mixture of E-5-(2-bromovinyl)-2'-deoxyuridine (2g, 6.01 mmol), N-tert. butoxycarbonylphenylalanine-N-hydroxy succinimide ester (2.4 g, 6.62 mmol) and 4-dimethylaminopyridine (0.4 g, 3.2 mmol) were stirred together in dry tetrahydrofuran (100 ml) at 75° for 3 hours. The solvent was removed under reduced pressure and the residue dissolved in dichloromethane (100 mL). The organic layer was washed with 1*M* hydrochloric acid (2 x 100 mL), cold saturated sodium hydrogen carbonate solution (2 x 100 mL) and water (100 mL) before drying ($Na_2SO_4$). The solvent was removed under reduced pressure affording a white foam. Thin layer chromatography of the foam on silica gel eluting with chloroform/methanol (20:1) showed the presence of three components ($R_f$ 0.60, 0.41, 0.21). The three components were separated by column chromatography on silica gel eluting with chloroform/methanol (50:1).

The following three compounds were isolated by this method.

| Example No. | Yield % | m.p.°C |
| --- | --- | --- |
| Example 1 | | |
| E-5-(2-Bromovinyl)-3',5'-di-O-(tert butoxycarbonylphenylalanyl)-2'-deoxyuridine. | 30 | foam |
| Example 2 | | |
| E-5-(2-bromovinyl)-3'-O-(tert butoxycarbonylphenylalanyl)-2'-deoxyuridine. | 16 | 98-102 |
| Example 3 | | |
| E-5-(2-Bromovinyl)-5'-O-(tert butoxycarbonylphenylalanyl)-2'-deoxyuridine | 32 | 114-6 |

Further characterising data for Example 1, 2 and 3 are as follows:

Example 1

E-5-(2-Bromovinyl)-3',5'-di-O-(tert. butoxycarbonylphenylalanyl)-2'-deoxyuridine

Tlc $R_f$ = 0.60 in chloroform/methanol (20:1)
$\nu_{max}$ (nujol) 3350 (NH), 1750, 1700 (C=O) $cm^{-1}$;
$^1$Hnmr [$(CD_3)_2SO$) $\delta$ 1.37 (18H, 2 x s, 6 x $CH_3$), 2.3 (2H, m, 2'$CH_2$), 2.94 (4H, m, 2 x Ph$CH_2$), 3.9 (1H, m, 4'CH), 4.21 (4H, m, 5'$CH_2$ + 2x NHCHC), 5.0 (1H, m, 3'CH), 6.07 (1H, m, 1'CH), 6.92 (1H, d, J = 14Hz, CH = CHBr), 7.3 (13H, m, aromatic + CH = CHBr + 2 x NHCH), 7.77 (1H, s, 6 - CH), 11.67 )1H, br.s, $D_2O$ exchangeable, NH);

Analysis

Found: C, 56.48; H, 5.58; N, 6.43. $C_{39}H_{47}N_4O_{11}Br$
requires: C, 56.59; H, 5.72; N, 6.77.

Example 2

E-5-(2-Bromovinyl)-3'-O-(tert.butoxycarbonylphenylalanyl)-2'-deoxyuridine

Tlc $R_f$ = 0.41 in chloroform/methanol (20:1). $\nu_{max}$(nujol) 3400, 3350 (NH,OH), br.1700 (C=O) $cm^{-1}$; $^1H$ nmr [$(CD_3)_2SO$] $\delta$ 1.4 (9H, s, $(CH_3)_3$), 2.26 (2H, m, 2'$CH_2$), 2.94 (2H, m, Ph $CH_2$), 3.7 (2H, m, 5'$CH_2$), 4.15 (2H, m, 4'CH + NHCHC(=O)), 5.21 (2H, m, $^1H$ $D_2O$ exchangeable, 3'CH + OH), 6.1 (1H, m, 1'CH), 6.8 (1H, d, J = 14Hz, CH = CHBr), 7.3 (7H, m, aromatic + CH=CHBr + NHCH), 8.03 (1H, s, 6-CH), 11.6 (1H, br.s, $D_2O$ exchangeable, NH).

Analysis

Found: C, 51.95; H, 5.01; N, 7.13. $C_{25}H_{30}N_3O_8Br$ requires: C, 51.73; H, 5.21; N, 7.24.

Example 3

E-5-(2-Bromovinyl)-5'-O-(tert.butoxycarbonylphenylalanyl)-2'-deoxyuridine

Tlc $R_f$ = 0.21 in chloroform/methanol (20:1).
$\nu_{max}$ (nujol) 3350 (OH, NH), br.1700 (C=O) $cm^{-1}$.
$^1$Hnmr $[(CD_3)_2SO]$ $\delta$ 1.37 (9H, s, $(CH_3)_3$), 2.14 (2H, m, 2'$CH_2$), 2.94 (2H, m, $Ph\underline{CH_2}$), 3.9 (1H, m, 4'CH), 4.2 (4H, m, 5'$CH_2$ + 3'CH + $NH\underline{CH}\underset{O}{\underset{\|}{C}}$), 5.37 (1H, d, J = 4.5Hz, $D_2O$ exchangeable, OH), 6.14 (1H, t, J = 6Hz, 1'CH), 6.94 (1H, d, J = 14Hz, $\underline{CH}$ = CHBr), 7.29 (7H, m, aromatic + CH = $\underline{CH}$Br + $CH\underline{NH}$), 7.77 (1H, s, 6 - CH), 11.6 (1H, br.s, $D_2O$ exchangeable, NH).

Analysis

Found: C, 52.05; H, 5.42; N, 7.05. $C_{25}H_{30}N_3O_8Br$ requires: C, 51.73; H, 5.21; N, 7.24.

Examples 4, 5 and 6

Preparation of E-5-(2-Bromovinyl)-2'-deoxyuridine-3'(5')-O- and 3',5'-di-O-(tert.butoxycarbonylglycyl)-esters

The three glycyl esters were prepared as for the preparation of the phenylalanyl esters (Examples 1, 2 and 3). The crude reaction residue was a yellow foam which was shown by tlc in chloroform/methanol (5:1) to be three components ($R_f$ 0.68, 0.57, 0.47). The three components were separated by column chromatography on silica gel eluting with chloroform/methanol (20:1).

The following three compounds were isolated by this method.

| Example No. | Yield % | m.p.°C |
|---|---|---|
| Example 4 | | |
| E-5-(2-Bromovinyl)-3',5'-di-O-(tert.butoxycarbonylglycyl)-2'-deoxyurdine. | 17 | foam |
| Example 5 | | |
| E-5-(2-Bromovinyl)-3'-O-(tert.-butoxycarbonylglycyl)-2'-deoxyuridine. | 4 | foam |
| Example 6 | | |
| E-5-(2-Bromovinyl)-5'-O-(tert.-butoxycarbonylglycyl)-2'-deoxyuridine. | 30 | 112-4 |

Example 5

E-5-(2-Bromovinyl)-3'-O-(tert.butoxycarbonylglycyl)-2'-deoxyuridine

Tlc $R_f$ = 0.57 in chloroform/methanol (5:1); $\nu_{max}$(nujol) 3350 (OH,NH), 1760, 1700 (C=O) $cm^{-1}$. $^1H$ n.m.r. [$(CD_3)_2SO$] $\delta$1.4 (9H, s, $(CH_3)_3$), 2.33 (2H, m, 2'$CH_2$), 3.69 (4H, m, 5'$CH_2$ + $CH_2$), 4.0 (1H, m, 4'CH), 5.27 (2H, m, 1H $D_2O$ exchangeable, 3'CH + OH), 6.16 (1H, t, J = 7.5Hz, 1'CH), 6.85 (1H, d, J = 14Hz, CH = CHBr), 7.23 (1H, br.s, NH$CH_2$), 7.26 (1H, d, J = 14Hz,CH=CHBr), 8.06 (1H, s, 6 - CH), 11.62 (1H, br.s, $D_2O$ exchangeable, NH);

Analysis

Found: C, 44.06; H, 4.87; N, 8.10. $C_{18}H_{24}N_3O_8Br$ requires: C, 44.09; H, 4.93; N, 8.57.

Example 5

E-5-(2-Bromovinyl)-3'-0-(tert.butoxycarbonylglycyl)-2'-deoxyuridine

Tlc $R_f$ = 0.57 in chloroform/methanol (5:1); $\nu_{max}$(nujol) 3350 (OH,NH), 1760, 1700 (C=O) $cm^{-1}$. $^1H$ n.m.r. [$(CD_3)_2SO$] $\delta$ 1.4 (9H, s, $(CH_3)_3$), 2.33 (2H, m, 2'$CH_2$), 3.69 (4H, m, 5'$CH_2$ + $CH_2$), 4.0 (1H, m, 4'CH), 5.27 (2H, m, 1H $D_2O$ exchangeable, 3'CH + OH), 6.16 (1H, t, J = 7.5Hz, 1'CH), 6.85 (1H, d, J = 14Hz, CH = CHBr), 7.23 (1H, br.s, NH$CH_2$), 7.26 (1H, d, J = 14Hz, CH=CHBr), 8.06 (1H, s, 6 - CH), 11.62 (1H, br.s, $D_2O$ exchangeable, NH);

Analysis

Found: C, 44.06; H, 4.87; N, 8.10. $C_{18}H_{24}N_3O_8Br$ requires: C, 44.09; H, 4.93; N, 8.57.

Example 6

E-5-(2-Bromovinyl)-5'-O-(tert.butoxycarbonylglycyl)-2'-deoxyuridine

Tlc $R_f$ = 0.47 in chloroform/methanol (5:1);
$\nu_{max}$ (nujol) 3500, 3350 (OH, NH), 1760, 1690 (C=O) $cm^{-1}$;
$^1$Hnmr [$(CD_3)_2SO$] $\delta$ 1.4 (9H, s, $(CH_3)_3$), 2.17 (2H, m, 2'$CH_2$), 3.68 (2H, d, J=6Hz, $CH_2$), 3.91 (1H, m, 4'CH), 4.22 (3H, m, 5'$CH_2$ + 3'CH), 5.35 (1H, br.s, $D_2O$ exchangeable, OH), 6.13 (1H, t, J=5Hz, 1'CH), 6.87 (1H, d, J=14Hz, CH=CHBr), 7.2 (1H, m, NHCH$_2$), 7.31 (1H, d, J=14Hz, CH=CHBr), 7.74 (1H, s, 6-CH), 11.6 (1H, br.s, $D_2O$ exchangeable, NH).

Analysis

Found: C, 44.20; H, 4.98; N, 8.31. $C_{18}H_{24}N_3O_8Br$ requires: C, 44.09; H, 4.93; N, 8.57.

Examples 7, 8 and 9

General method for the trifluoroacetic acid deprotection of E-5-(2-Bromovinyl)-2'-deoxyuridine-3'-(5')-O- and 3',5'-di-O-(tert.butoxycarbonylphenylalanyl)esters

A solution of E-5-(2-bromovinyl)-2'-deoxyuridine-3'-(5')-O- or 3',5'-di-O-(tert.butoxycarbonylphenyl-alanyl) esters (0.43 mmol) in trifluoroacetic acid ( 3 mL) was stirred at 25° for 15 minutes. The solvent was removed *in vacuo* and the oily residue triturated with dry ether to give a white solid.

The following three compounds were prepared by this method.

Example No.

| | Yield % |
|---|---|
| Example 7<br>E-5-(2-Bromovinyl)-3',5'-di-O-phenylalanyl-2'-deoxyuridine bis trifluoroacetate | 79 |
| Example 8<br>E-5-(2-Bromovinyl)-3'-O-phenylalanyl-2'-deoxyuridine trifluoroacetate | 91 |
| Example 9<br>E-5-(2-Bromovinyl)-5'-O-phenylalanyl-2'-deoxyuridine trifluoroacetate | 94 |

Further characterising data for Examples 7, 8 and 9 are as follows.

Example 7

E-5-(2-Bromovinyl)-3',5'-di-O-phenylalanyl-2'-deoxyuridine bis trifluoroacetate

$^1$Hnmr [$(CD_3)_2SO$] δ 2.3 (2H, m, 2'$CH_2$), 3.18 (4H, m, 2 x Ph$CH_2$), 3.86 (1H, m, 4'CH), 4.3 (4H, m, 5'$CH_2$ + 2xCH), 5.05 (1H, m, 3'CH), 6.05 (1H, t, J=7.5Hz, 1'CH), 6.9 (1H, d, J=14Hz, CH=CHBr), 7.35 (11H, m, aromatic + CH=CHBr), 7.8 (1H, s, 6-CH), 8.67 (6H, br.s, $D_2O$ exchangeable, 2 x $\overset{\oplus}{N}H_3$), 11.5 (1H, br.s, $D_2O$ exchangeable, NH).

Analysis

Found: C, 46.54; H, 3.77; N, 6.24. $C_{33}H_{33}N_4BrF_6O_{11}$ requires: C, 46.33; H, 3.89; N, 6.55.

Example 8

E-5-(2-Bromovinyl)-3'-O-phenylalanyl-2'-deoxyuridine trifluoroacetate

$^1$Hnmr [$(CD_3)_2SO$] δ 2.28 (2H, m, 2'$CH_2$), 3.08 (2H, m, Ph$CH_2$), 3.66 (2H, m, 5'$CH_2$), 4.0 (1H, m, 4'CH), 4.32 (1H, t, J=7.5Hz, $PhCH_2CH$), 5.24 (2H, m, 1H $D_2O$ exchangeable, 3'CH+OH), 6.06 (1H, t, J=7.5Hz, 1'CH), 6.84 (1H, d, J=14Hz, CH=CHBr), 7.3 (6H, m, aromatic + CH=CHBr), 8.06 (1H, s, 6-CH), 8.67 (3H, br.s, $D_2O$ exchangeable, $\overset{+}{N}H_3$), 11.66 (1H, br.s, $D_2O$ exchangeable, NH).

Analysis

Found: C, 44.29; H, 3.67; N, 6.82. $C_{22}H_{23}N_3BrF_3O_8$ requires: C, 44.46; H, 3.90; N, 7.07.

Example 9

E-5-(2-Bromovinyl)-5'-O-phenylalanyl-2'-deoxyuridine trifluoroacetate

$^1$Hnmr [$(CD_3)_2SO$] $\delta$ 2.2 (2H, m, 2'$CH_2$), 3.13 (2H, m, Ph$\underline{CH_2}$), 3.9 (1H, m, 4'CH), 4.2 (4H, m, 5'$CH_2$+3'CH+Ph$CH_2\underline{CH}$), 6.19 (1H, t, J=7.5Hz, 1'CH), 6.96 (1H, t, J=14Hz, $\underline{CH}$=CHBr), 7.3 (6H, m, aromatic+CH=$\underline{CH}$Br), 7.8 (1H, s, 6-CH), 8.62 (3H, br.s, $D_2O$ exchangeable, $\overset{\oplus}{N}H_3$), 11.7 (1H, br.s, $D_2O$ exchangeable, NH).

Analysis

Found: C, 44.21; H, 3.85; N, 6.79. $C_{22}H_{23}N_3BrF_3O_8$ requires: C, 44.46; H, 3.90; N, 7.07.

0080305

Example 10

Method for the sulphuric acid deprotection of E-5-(2-Bromovinyl)-3',5'-di-O-(tert.butoxycarbonyl-phenylalanyl)-2'-deoxyuridine

A solution of E-5-(2-bromovinyl)-3',5'-di-O-(tert butoxycarbonylphenylalanyl)-2'-deoxyuridine (100 mg, 0.12 mmol) in dry ether (10 mL) was treated with 98% sulphuric acid (14 µL, 0.26 mmol) and stirred at 25° for 1 hour. The solvent was removed *in vacuo* and the residue stirred vigorously with ether (20 mL). The white solid was quickly filtered and dried.

$^1$Hnmr [$(CD_3)_2SO$] $\delta$ 2.32 (2H, m, 2'$CH_2$), 3.1 (4H, m, 2 x Ph$CH_2$), 3.87 (1H, m, 4'CH), 4.25 (4H, m, 5'$CH_2$ + 2 x $PhCH_2$CH), 5.0 (1H, m, 3'CH), 6.03 (1H, t, J=7.5Hz, 1'CH), 6.9 (1H, d, J=14Hz, CH=CHBr), 7.26 (11H, m, aromatic + CH=CHBr), 7.8 (1H, s, 6-CH), 8.47 (6H, br.s, $D_2O$ exchangeable, 2 x $\overset{\oplus}{N}H_3$), 11.7 (1H, br.s, $D_2O$ exchangeable, NH).

Analysis

Found: C, 41.91; H, 4.61; N, 6.29; S, 7.69.
$C_{29}H_{35}N_4BrO_{15}S_2$
requires: C, 42.29; H, 4.28; N, 6.80; S, 7.79.

Examples 11 and 12

General Method for the Preparation of 3',5'-di-(t-butoxycarbonylaminoacyl)esters of E-5-(2-bromovinyl)-2'-deoxyuridine

E-5-(2-Bromovinyl)-2'-deoxyuridine (2 g, 6 mmol), N-t-butoxycarbonylamino acid-N-hydroxysuccinimide ester (5 g, 15 mmol) and 4-dimethylaminopyridine (0.4 g, 3.3 mmol) were stirred in dry tetrahydrofuran (150 mL) at 80° for 24 hours. The solvent was removed under reduced pressure and the residue dissolved in chloroform (100 mL). The organic layer was washed with 1M hydrochloric acid (2 x 100 mL), cold saturaed sodium bicarbonate solution (2 x 100 mL) and water (100 mL) before drying ($Na_2SO_4$). The 3',5'-di-O-(t-butoxycarbonylaminoacyl) esters were normally isolated by column chromatography on silica gel.

Example 11

E-5-(2-Bromovinyl)-3',5'-di(t-butoxycarbonyl-p-fluoro-DL-phenylalanyl)-2'-deoxyuridine

Isolated as a white foam in 89% yield by column chromatography on silica gel eluting with chloroform/methanol (50:1). Tlc Rf = 0.55 in chloroform/methanol (30:1);

$\nu$max (nujol) 3350 (NH), 1740, 1710 (C=O) $cm^{-1}$;
$^1H$ nmr [$(CD_3)_2SO$] $\delta$ 1.35 (18H, s, $(CH_3)_6$), 2.33 (2H, m, 2'-$CH_2$), 2.95 (4H, m, 2xPh$\underline{CH_2}$), 3.9-4.45 (5H, m, 4'-CH+5'-$CH_2$+2x$\underline{CH}$NH), 5.16 (1H, m, 3'-CH), 6.15 (1H, t, J=6Hz), 6.93 (1H, d, J=14Hz, $\underline{CH}$=CHBr), 7.0-7.6 (11H, m, aromatic + 2x$\underline{NH}$CH+CH=$\underline{CH}$Br), 7.83 (1H, s, 6-CH), 11.74 (1H, s, $D_2O$ exchangeable, 3-NH).

Analysis
Found: C, 54.36; H, 5.23; N, 6.52% $C_{39}H_{45}BrF_2N_4O_{11}$ requires: C, 54.23; H, 5.25; N, 6.49%.

0080305

Example 12

E-5-(2-Bromovinyl)-3',5'-di(t-butoxycarbonyl-L-leucyl)-2'-deoxyuridine

Isolated as a white foam in 41% yield by column chromatography on silica gel eluting wtih chloroform/methanol (50:1) and then a second column eluting with chloroform. Rf = 0.42 in chloroform/methanol (40:1);

$\nu$max (nujol) 3350 (NH), 1745, 1700 (C=O) $cm^{-1}$; $^1H$ nmr [$(CD_3)_2SO$] $\delta$ 0.9 (12H, m, 2x($\underline{CH_3}$)$_2$CH), 1.4 (18H, s, 2x($\underline{CH_3}$)$_3$CO), 1.5 (6H, m, 2x$\underline{CH_2CH}$($CH_3$)$_2$), 2.42 (2H, m, 2'-$CH_2$), 3.98 (2H, m, 2x$\underline{CH}$NH), 4.2 (3H, m, 5'-$CH_2$ + 4'-CH), 5.21 (1H, m, 3'-CH), 6.18 (1H, t, J=6Hz, 1'-CH), 6.91 (1H, d, J=14Hz, $\underline{CH}$=CHBr), 7.35 (1H, d, J=14Hz, CH=$\underline{CH}$Br), 7.4 (2H, br.m, 2x$\underline{NH}$CH), 7.82 (1H, s, 6-CH), 11.7 (1H, s, $D_2O$ exchangeable, 3-NH).

Analysis

Found: C, 52.26; H, 6.69; N, 7.41% $C_{33}H_{51}BrN_4O_{11}$ requires: C, 52.17; H, 6.77; N, 7.38%.

Examples 13 to 16

The general deprotection reaction method of Examples 7 to 9 was used to prepared these Examples, with the following characterising data.

Example 13

E-5-(2-Bromovinyl)-5'-glycyl-2'-deoxyuridine trifluoroacetate

Isolated in 88% yield.

$\nu$max (nujol) 1760, 1720, 1680 $cm^{-1}$;
$^1H$ nmr [$(CD_3)_2SO$] $\delta$ 2.27 (2H, m, 2'-$CH_2$), 3.85 (2H, m, $\underline{CH_2}NH_3$), 4.1-4.8 (4H, m, 5'-$CH_2$ + 3'-CH + 4'-CH), 5.95 (1H, br.s, $D_2O$ exchangeable, OH), 6.2 (1H, t, J=6Hz, 1'-CH), 6.94 (1H, d, J=14Hz, $\underline{CH}$=CHBr), 7.4 (1H, d, J=14Hz, CH=$\underline{CH}$Br), 7.8 (1H, s, 6-CH), 8.3 (3H, br.s, $D_2O$ exchangeable, $NH_3$), 11.6 (1H, s, $D_2O$ exchangeable, 3-NH).

Analysis
Found: C, 36.65; H, 3.48; N, 7.94% $C_{15}H_{17}BrF_3N_3O_8$ requires: C, 35.73; H, 3.40; N, 8.33%.

0080305

Example 14

E-5-(2-Bromovinyl)-3',5'-di-glycyl-2'-deoxyuridine bis trifluoroacetate

Isolated in 96% yield.

$\nu$max (nujol) 1760, 1720, 1680 $cm^{-1}$;
$^1$H nmr [$(CD_3)_2SO$] $\delta$ 2.51 (2H, m, 2'-$CH_2$), 3.9 (4H, s, 2x$CH_2$), 4.2-4.7 (3H, m, 5'-$CH_2$ + 4'-CH), 5.4 (1H, m, 3'-CH), 6.24 (1H, t, J=6Hz, 1'-CH), 6.93 (1H, d, J=14Hz, <u>CH</u>=CHBr), 7.4 (1H, d, J=14Hz, CH=<u>CH</u>Br), 8.52 (7H, br.s, $D_2O$ exchangeable, 2 x $NH_3$ + NH).

Analysis
Found: C, 34.21; H, 2.91; N, 8.08% $C_{19}H_{21}BrF_6N_4O_{11}$ requires: C, 33.79; H, 3.13; N. 8.30%.

Example 15

E-5-(2-Bromovinyl)-3',5'-di-(p-fluoro-DL-phenylalanyl)-2'-deoxyuridine bis trifluoroacetate

Isolated in 94% yield.

$\upsilon$max (nujol) 1755, 1710, 1680 $cm^{-1}$;
$^1H$ nmr [$(CD_3)_2SO$] $\delta$ 2.3 (2H, m, 2'-$CH_2$), 3.1 (4H, m, 2xPh$\underline{CH_2}$), 3.7-4.6 (5H, m, 4'-CH + 5'-$CH_2$ + 2x$\underline{CH}CH_2$), 5.23 (1H, m, 3'-CH), 6.13 (1H, t, J=6Hz, 1'-CH), 6.8-7.5 (10H, m, aromatic + CH=CHBr), 7.9 (1H, s, 6-CH), 8.7 (7H, br.s, $D_2O$ exchangeable, 2x$NH_3$ + NH).

Analysis
Found: C, 44.94; H, 3.56; N. 6.42% $C_{33}H_{31}BrF_8N_4O_{11}$
requires: C, 44.46; H, 3.51; N, 6.28%.

Example 16

E-5-(2-Bromovinyl)-3',5'-di-L-leucyl-2'-deoxyuridine trifluoroacetate

Isolated in 87% yield.

$\nu$max (nujol) 1760, 1730, 1680 $cm^{-1}$;
$^1H$ nmr [$(CD_3)_2SO$] $\delta$ 0.9 (12H, m, $4xCH_3$), 1.64 (6H, m, $2x\underline{CH_2CH}(CH_3)_2$), 2.45 (2H, m, 2'-$CH_2$), 4.0 (2H, m, 2x$\underline{CH}$NH), 4.38 (3H, m, 5'-$CH_2$ + 4'-CH), 5.35 (1H, m, 3'-CH), 6.24 (1H, t, J=14Hz, CH=$\underline{CH}$Br), 6.88 (1H, d, J=14Hz, $\underline{CH}$=CHBr), 7.34 (1H, d, J=14Hz, CH=$\underline{CH}$Br), 7.9 (1H, s, 6-CH), 8.65 (7H, br.s, $D_2O$ exchangeable, $2xNH_3$ + NH).

Analysis
Found: C, 40.63; H, 4.95; N. 7.00% $C_{27}H_{37}BrF_6N_4O_{11}$
requires: C, 41.18; H, 4.74; N, 7.11%.

Example 17

E-5-(2-Bromovinyl)-3',5'-di-glycyl-2'-deoxyuridine bis bisulphate

A solution of E-5-(2-bromovinyl)-3',5'-di-(t-butoxycarbonylglycyl)-2'-deoxyuridine (Example 4) (323 mg, 0.5 mmol) in dry ether (5 mL) was treated with 98% sulphuric acid (57 μL, 1.07 mmol) and stirred at 0° for 15 minutes. The deposited solid was filtered off, washed well with ether and dried to give E-5-(2-bromovinyl)-3',5'-di-glycyl-2'-deoxyuridine bis bisulphate in 31% yield.

$\nu$max (nujol) 1760, 1720, 1680 $cm^{-1}$;
$^1H$ nmr [$(CD_3)_2SO$] $\delta$ 2.5 (2H, m, 2'-$CH_2$), 3.89 (4H, m, 2x$CH_2$), 4.4 (3H, m, 5'-$CH_2$ + 4'-CH), 5.39 (1H, m, 3'-CH), 6.21 (1H, t, J=6Hz, 1'-CH), 6.89 (1H, d, J=14Hz, CH=CHBr), 7.38 (1H, d, J=14Hz, CH=CHBr), 7.9 (1H, s, 6-CH), 8.3-8.68 (9H, m, $D_2O$ exchangeable, 2 x $NH_3$ + $2HSO_4^-$ + NH).

Analysis
Found: C, 28.19; H, 3.76; N, 7.99% $C_{15}H_{23}BrN_4O_{15}S_2$ requires: C, 28.00; H, 3.60; H, 8.71%.

Example 16

E-5-(2-Bromovinyl)-3',5'-di-L-leucyl-2'-deoxyuridine bis bisulphate

A solution of E-5-(2-bromovinyl)-3',5'-di-(t-butoxycarbonyl-L-leucyl)-2'-deoxyuridine (Example 12) (250mg, 0.3mmol) in dry ether (10mL) was treated with 98% sulphuric acid (36μL, 0.66mmol) and stirred at $0^{o}$ for 15 minutes. The solvents were removed under reduced pressure and the residual foam triturated well with ether to give E-5-(2-bromovinyl)-3',5'-di-L-leucyl-2'-deoxyuridine bis bisulphate salt as a white solid in 99% yield. νmax (nujol) 1760, 1670-1730, 1600 $cm^{-1}$; $^{1}H$ nmr [$(CD_3)_2SO$] δ 0.9 (12H, m, 4 x $CH_3$), 1.67 (6H, m, 2 x $CH_2CH$), 2.5 (2H, m, 2'$CH_2$), 4.0 (2H, m, 2 x $C\underline{H}\overset{+}{N}H_3$), 4.28-4.65 (3H, m, 5'$CH_2$ + 4'CH), 5.39 (1H, m, 3'CH), 6.28 (1H, t, J = 6Hz, 1'CH), 6.95 (1H, d, J = 14Hz, $\underline{CH}$=CHBr), 7.49 (1H, d, J = 14Hz, CH=$\underline{CH}$Br), 7.65 (2H, br.s, $D_2O$ exchangeable, $2HSO_4^-$), 7.91 (1H, s, 6-CH), 8.39 (6H, br.s, $D_2O$ exchangeable, 2 x $\overset{+}{N}H_3$), 11.78 (1H, s, $D_2O$ exchangeable, 3-NH).

Analysis

Found: C, 36.87; H, 5.40; N, 6.92 %. $C_{23}H_{39}BrN_4O_{15}S_2$ requires C, 36.56; H, 5.20; N, 7.42 %.

BAD ORIGINAL

General Method for the Preparation of 3',5'-Di-(t-butoxycarbonylaminoacyl) esters of E-5-(2-Bromovinyl)-2'-deoxyuridine (Examples 19 - 23).

E-5-(2-Bromovinyl)-2'-deoxyuridine (2g, 6mmol), N-t-butoxycarbonylamino acid-N-hydroxysuccinimide ester (≃5g, 15mmol) and 4-dimethylaminopyridine (1.83g, 15mmol) were stirred in dry tetrahydrofuran (200mL) at 80$^{o}$ for 24 hours. The solvent was removed under reduced pressure and the residue dissolved in chloroform (200mL). The organic layer was washed with 1M hydrochloric acid (2 x 150mL) cold saturated sodium bicarbonate solution (2 x 150mL) and water (150mL) before drying ($Na_2SO_4$). The solvent was removed under reduced pressure affording a foam. The 3',5'-di-(t-butoxycarbonylaminoacyl) esters were isolated by column chromatography on silica gel.

The following compounds were isolated by this method:

| Example No. | Yield % |
|---|---|
| Example 19<br>E-5-(2-Bromovinyl)-3',5'-di-(t-butoxycarbonyl-D-leucyl)-2'-deoxyuridine | 78 |
| Example 20<br>E-5-(2-Bromovinyl)-3',5'-di-(t-butoxycarbonyl-D-phenylalanyl)-2'-deoxyuridine | 50 |
| Example 21<br>E-5-(2-Bromovinyl)-3',5'-di-(t-butoxycarbonyl-L-methionyl)-2'-deoxyuridine | 59 |
| Example 22<br>E-5-(2-Bromovinyl)-3',5'-di-(t-butoxycarbonyl-L-valyl)-2'-deoxyuridine | 51 |
| Example 23<br>E-5-(2-Bromovinyl)-3',5'-di-(t-butoxycarbonyl-L-isoleucyl)-2'-deoxyuridine | 36 |

BAD ORIGINAL

Example 19

E-5-(2-Bromovinyl)-3',5'-di-(t-butoxycarbonyl-D-leucyl)-2'-deoxyuridine

Tlc Rf = 0.58 in ethyl acetate/hexane (2:1); νmax (nujol) 3350, 1700-1750 (broad) $cm^{-1}$; $^1H$ nmr $[(CD_3)_2SO]$ δ 0.85 (12H, m, 4 x $CH_3$), 1.37 (24H, m, 2 x $C(CH_3)_3$ + 2 x $CH_2CH$), 2.37 (2H, m, 2'$CH_2$), 3.93 (2H, m, 2 x $C\underline{H}NH$), 4.14 (1H, m, 4'CH), 4.27 (2H, m, 5'$CH_2$), 5.19 (1H, m, 3'CH), 6.14 (1H, t, J = 6Hz, 1'CH), 6.88 (1H, d, J = 14Hz, $C\underline{H}$=CHBr), 7.29 (1H, d, J = 14Hz, CH=$C\underline{H}$Br), 7.3 (2H, br.m, 2 x $\underline{NH}\overset{O}{\overset{\|}{C}}OC(CH_3)_3$), 7.8 (1H, s, 6-CH), 11.66 (1H, s, $D_2O$ exchangeable, 3-NH).

Analysis

Found: C, 52.39; H, 6.81; N, 7.37 %. $C_{33}H_{51}BrN_4O_{11}$ requires C, 52.17; H, 6.77; N, 7.38 %.

Example 20

E-5-(2-Bromovinyl)-3',5'-di-(t-butoxycarbonyl-D-phenylalanyl)-2'-deoxyuridine

Tlc Rf = 0.26 in ethyl acetate/hexane (2:3); νmax (nujol) 3340, 1700-1750 (broad) $cm^{-1}$; $^1H$ nmr $[(CD_3)_2SO]$ δ 1.27 (18H, s, 6 x $CH_3$), 2.2 (2H, m, 2'$CH_2$), 2.9 (4H, m, 2 x $Ph\underline{CH_2}$), 3.9-4.3 (5H, m, 5'$CH_2$ + 4'CH + 2 x $CHCH_2$), 5.1 (1H, m, 3'CH), 6.02 (1H, t, J = 6Hz, 1'CH), 6.88 (1H, d, J = 14Hz, $\underline{CH}$=CHBr), 7.26 (13H, m, aromatic + CH=$\underline{CH}$Br + 2 x $\underline{NH}\overset{O}{\overset{\|}{C}}OC(CH_3)_3$), 7.78 (1H, s, 6-CH), 11.68 (1H, br.s, $D_2O$ exchangeable, 3-NH).

Analysis

Found: C, 56.51; H, 5.65; N, 6.49 %. $C_{39}H_{47}BrN_4O_{11}$ requires C, 56.59; H, 5.72; N, 6.76 %.

0080305

Example 21

E-5-(2-Bromovinyl)-3',5'-di-(t-butoxycarbonyl-L-methionyl)-2'-deoxyuridine

Tlc Rf = 0.31 in chloroform/methanol (50:1); νmax (nujol) 3340, 1680-1750 (broad), 1600 $cm^{-1}$; $^1H$ nmr [$(CD_3)_2SO$] δ 1.38 (18H, s, 2 x $C(CH_3)_3$), 1.9 (4H, m, 2 x $\underline{CH_2}CH$), 2.02 (3H, s, $SCH_3$), 2.05 (3H, s, $SCH_3$), 3.95-4.4 (5H, m, 5'$CH_2$ + 4'CH + 2 x $\underline{CH}NH$), 5.26 (1H, m, 3'CH), 6.19 (1H, t, J = 6Hz, 1'CH), 6.9 (1H, d, J = 14Hz, $\underline{CH}$=CHBr), 7.34 (1H, d, J = 14Hz, CH=$\underline{CH}$Br), 7.4 (2H, m, 2 x $\underline{NH}\overset{O}{\overset{\|}{C}}OC(CH_3)_3$), 7.82 (1H, s, 6-CH), 11.7 (1H, s, $D_2O$ exchangeable, 3-NH).

Analysis

Found: C, 47.40; H, 5.61; N, 6.83 %. $C_{31}H_{47}BrN_4O_{11}S_2$ requires C, 46.79; H, 5.95; N, 7.04 %.

Example 22

E-5-(2-Bromovinyl)-3',5'-di-(t-butoxycarbonyl-L-valyl)-2'-deoxyuridine

Tlc Rf = 0.31 in ethyl acetate/hexane (1:2); νmax (nujol) 3350, 1700-1750 (broad), 1600 $cm^{-1}$; $^1H$ nmr [$(CD_3)_2SO$] δ 0.87 (12H, m, 4 x $CH_3$), 1.35 (18H, s, 2 x $C(CH_3)_3$), 1.96 (2H, m, 2 x $\underline{CH}(CH_3)_2$), 2.39 (2H, m, 2'$CH_2$), 3.78 (2H, m, 2 x $\underline{CH}NH$), 4.18 (1H, m, 4'CH), 4.27 (2H, m, 5'$CH_2$), 5.22 (1H, m, 3'CH), 6.19 (1H, t, J = 6Hz, 1'CH), 6.89 (1H, d, J = 14Hz, $\underline{CH}$=CHBr), 7.2 (2H, m, 2 x $\underline{NH}\overset{O}{\overset{\|}{C}}OC(CH_3)_2$), 7.3 (1H, d, J = 14Hz, CH=$\underline{CH}$Br), 7.8 (1H, s, 6-CH), 11.7 (1H, s, $D_2O$ exchangeable, 3-NH).

Analysis

Found: C, 51.08; H, 6.17; N, 7.57 %. $C_{31}H_{47}BrN_4O_{11}$ requires C, 50.89; H, 6.48; N, 7.66 %.

BAD ORIGINAL

Example 23

E-5-(2-Bromovinyl)-3',5'-di-(t-butoxycarbonyl-L-isoleucyl)-2'-deoxyuridine

Tlc Rf = 0.26 in chloroform/methanol (50:1); νmax (nujol) 3350, 1700-1750 (broad), 1600 $cm^{-1}$; $^1H$ nmr [$(CD_3)_2SO$] δ 0.8 (12H, m, 4 x $CH_3$), 1.2-2.0 (24H, m, 2 x $C(CH_3)_3$ + 2 x $CH_2$ + 2 x $\underline{CH}CH_3$), 2.31 (2H, m, 2'$CH_2$), 3.8 (2H, m, 2 x $\underline{CH}NH$), 4.1-4.4 (3H, m, 5'$CH_2$ + 4'CH), 6.1 (1H, t, J = 6Hz, 1'CH), 6.8 (1H, d, J = 14Hz, $\underline{CH}$=CHBr), 7.2 (2H, m, 2 x $\underline{NH}\overset{O}{C}OC(CH_3)_3$), 7.24 (1H, d, J = 14Hz, CH=$\underline{CH}$Br), 7.73 (1H, s, 6-CH), 11.66 (1H, s, 3-NH).

Analysis

Found: C, 51.93; H, 6.64; N, 7.32 %. $C_{33}H_{51}BrN_4O_{11}$ requires C, 52.17; H, 6.77; N, 7.33 %.

BAD ORIGINAL

General method for the Preparation of 3',5'-Di-(t-butoxycarbonylaminoacyl) esters of E-5-(2-Bromovinyl)-2'-deoxyuridine (Examples 24 - 26)

E-5-(2-Bromovinyl)-2'-deoxyuridine (2g, 6mmol), N-t-butoxycarbonylamino acid p-nitrophenyl ester (≈5g, 15mmol) and 4-dimethylaminopyridine (1.83g, 15mmol) were stirred together in dry tetrahydrofuran (200mL) at 80$^{o}$ for 24 hours. The solvent was removed under reduced pressure and the residue dissolved in chloroform (200mL). The organic layer was washed with 1M hydrochloric acid (2 x 150mL), cold saturated sodium bicarbonate solution (2 x 150mL) and water (150mL) before drying ($Na_2SO_4$). The solvent was removed under reduced pressure affording a foam. The 3',5'-di-(t-butoxycarbonylaminoacyl) esters were isolated by column chromatography on silica gel.

The following compounds were isolated by this method.

| Example No. | Yield % |
| --- | --- |
| Example 24<br>E-5-(2-Bromovinyl)-3',5'-di-(t-butoxycarbonyl-L-alanyl)-2'-deoxyuridine | 56 |
| Example 25<br>E-5-(2-Bromovinyl)-3',5'-di-(t-butoxycarbonyl-L-glutaminyl)-2'-deoxyuridine | 14 |
| Example 26<br>E-5-(2-Bromovinyl)-3',5'-bis-($N_\beta$,$N_\varepsilon$-di-t-butoxycarbonyl-L-lysyl)-2'-deoxyuridine | 51 |

0080305

Example 24

E-5-(2-Bromovinyl)-3',5'-di-(t-butoxycarbonyl-L-alanyl)-2'-deoxyuridine

Tlc Rf = 0.47 in chloroform/methanol (10:1); νmax (nujol) 3330 (NH), 1750, 1700 (C=O) $cm^{-1}$; $^1H$ nmr [$(CD_3)_2SO$] δ 1.2-1.4 (24H, m, 2 x $C(CH_3)_3$ + 2 x $CH_3$), 2.40 (2H, m, 2'$CH_2$), 3.84-4.37 (5H, m, 5'$CH_2$ + 4'CH + 2 x CHNH), 5.22 (1H, m, 3'CH), 6.15 (1H, t, J = 6Hz, 1'CH), 6.86 (1H, d, J = 14Hz, CH=CHBr), 7.3 (2H, m, 2 x NH$\overset{O}{C}$), 7.3 (1H, d, J = 14Hz, CH=CHBr), 7.76 (1H, s, 6-CH), 11.66 (1H, br.s, $D_2O$ exchangeable, 3-NH).

Analysis

Found: C, 48.59; H, 5.78; N, 8.15 %. $C_{27}H_{39}N_4O_{11}Br$ requires C, 48.01; H, 5.82; N, 8.29 %.

Example 25

E-5-(2-Bromovinyl)-3',5'-di-(t-butoxycarbonyl-L-glutaminyl)-2'-deoxyuridine

Tlc Rf = 0.15 in chloroform/methanol (10:1); νmax (nujol) 3380, 3350, 3250, 3200 (NH), 1730, 1720, 1690, 1675, 1660 (C=O) $cm^{-1}$; $^1H$ nmr [$(CD_3)_2SO$] δ 1.4 (18H, m, 2 x $C(CH_3)_3$ ), 1.87 (4H, m, 2 x $CH_2CH$), 2.12 (4H, m, 2 x $CH_2\overset{O}{C}NH_2$), 2.42 (2H, m, 2'$CH_2$), 3.95 (2H, m, 2 x CHNH), 4.24 (3H, m, 5'$CH_2$ + 4'CH), 5.25 (1H, m, 3'CH), 6.2 (1H, t, J = 6Hz, 1'CH), 6.73 (2H, br.s, 2 x NH$\overset{O}{C}$-O$C(CH_3)_3$), 6.9 (1H, d, J = 14Hz, CH=CHBr), 7.27 (4H, m, 2 x $\overset{O}{C}NH_2$), 7.34 (1H, d, J = 14Hz, CH=CHBr), 7.8 (1H, s, 6-CH), 11.74 (1H, s, $D_2O$ exchangeable, 3-NH).

Analysis

Found: C, 47.25; H, 5.72; N, 10.56 %. $C_{31}H_{45}BrN_6O_{13}$ requires C, 47.15; H, 5.74; N, 10.64 %.

BAD ORIGINAL

Example 36

E-5-(2-Bromovinyl)-3',5'-bis-($N_\alpha$,$N_\varepsilon$-di-t-butoxycarbonyl-L-lysyl)-2'-deoxyuridine

Tlc Rf = 0.31 in chloroform/methanol (20:1); νmax (nujol) 3350 (NH), 1730, 1700 (C=) $cm^{-1}$; $^1H$ nmr [$(CD_3)_2SO$] δ 1.3-1.75 (48H, m, 4 x $C(CH_3)_3$ + 6 x $CH_2$), 2.33 (2H, m, 2'$CH_2$), 3.86 (2H, m, 2 x CHNH), 4.18 (3H, m, 5'$CH_2$ + 4'CH), 5.19 (1H, m, 3'CH), 6.15 (1H, t, J = 6Hz, 1'CH), 6.65 (2H, m, 2 x $NH\overset{O}{C}$), 6.87 (1H, d, J = 14Hz, CH=CHBr), 7.2 (2H, m, 2 x $NH\overset{O}{C}$), 7.3 (1H, d, J = 14Hz, CH=CHBr), 7.76 (1H, s, 6-CH), 11.65 (1H, br.s, $D_2O$ exchangeable, 3-NH).

Analysis

Found: C, 51.88; H, 7.01; N, 8.22 %. $C_{43}H_{69}N_6O_{15}Br$ requires C, 52.17; H, 7.03; N, 8.49 %.

Examples 27, 28 and 29

Reaction of E-5-(2-bromovinyl)-2'-deoxyuridine with N-t-butoxycarbonyl-L-proline N-hydroxysuccinimide ester was carried out under conditions identical to those described for the preparation of Examples 19 - 23. The following three products were isolated by column chromatography on silica gel, eluting with chloroform/methanol (50:1).

Example 27

E-5-(2-Bromovinyl)-3',5'-di-(t-butoxycarbonyl-L-prolyl)-2'-deoxyuridine

Isolated in 43% yield. Tlc Rf = 0.49 in chloroform/methanol (20:1); νmax (nujol) 1690-1740 (broad), 1755, 1600 $cm^{-1}$; $^1H$ nmr [$(CD_3)_2SO$] δ 1.38 (18H, m, 2 x $C(CH_3)_3$), 1.85 (8H, m, 4 x $CH_2$), 2.33 (2H, m, 2'$CH_2$), 3.3 (4H, m, 2 x $CH_2$-N), 4.0-4.4 (5H, m, 4'CH + 5'$CH_2$ + 2 x CHNH), 5.3 (1H, m, 3'CH), 6.2 (1H, t, J = 6Hz, 1'CH), 6.94 (1H, d, J = 14Hz, CH=CHBr), 7.4 (1H, d, J = 14Hz, CH=CHBr), 7.87 (1H, s, 6-CH), 11.73 (1H, s, $D_2O$ exchangeable, 3-NH).

Analysis

Found: C, 51.25; H, 5.14; N, 7.48 %. $C_{31}H_{43}BrN_4O_{11}$ requires C, 51.17; H, 5.96; N, 7.70 %.

Example 28

E-5-(2-Bromovinyl)-5'-(5-butoxycarbonyl-L-prolyl)-2'-deoxyuridine

Isolated in 8% yield. Tlc Rf = 0.17 in chloroform/methanol (20:1); νmax (nujol) 3400, 1680-1750 (broad), 1600 $cm^{-1}$; $^1H$ nmr [$(CD_3)_2SO$] δ 1.35 (9H, m, $C(CH_3)_3$), 1.72 (4H, m, 2 x $CH_2$), 2.13 (2H, m, 2'$CH_2$), 3.24 (2H, t, J = 6Hz, $CH_2$-N), 3.9-4.3 (5H, m, 5'$CH_2$ + 4'CH + 3'CH + CHN), 6.13 (1H, t, J = 6Hz, 1'CH), 6.9 (1H, d, J = 14Hz, CH=CHBr), 7.3 (1H, d, J = 14Hz, CH=CHBr), 7.75 (1H, s, 6-CH), 11.64 (1H, s, $D_2O$ exchangeable, 3-NH).

Cont'd./....

BAD ORIGINAL

Analysis
Found: C, 47.58; H, 4.87; N, 7.61 %. $C_{21}H_{28}BrN_3O_8$ requires C, 47.56; H, 5.32; N, 7.92 %.

Example 29

E-5-(2-Bromovinyl)-3'-(t-butoxycarbonyl-L-prolyl)-2'-deoxyuridine

Isolated in 9% yield. Tlc Rf = 0.33 in chloroform/methanol (20:1); νmax (nujol) 3400, 1680-1750 (broad), 1600 $cm^{-1}$; $^1H$ nmr [$(CD_3)_2SO$] δ 0.4 (9H, m, $C(CH_3)_3$), 1.85 (4H, m, 2 x $CH_2$), 2.36 (2H, m, 2'$CH_2$), 3.34 (2H, m, $CH_2$-N), 3.64 (2H, m, 5'$CH_2$), 3.9-4.3 (2H, m, 4'CH + 2 x CHN), 5.28 (1H, m, 3'CH), 6.18 (1H, t, J = 6Hz, 1'CH), 6.86 (1H, d, J = 14Hz, CH=CHBr), 7.3 (1H, d, J = 14Hz, CH=CHBr), 8.1 (1H, s, 6-CH), 11.63 (1H, s, $D_2O$ exchangeable, 3-NH).

Analysis
Found: C, 47.16; H, 5.36; N, 7.52 %. $C_{21}H_{28}BrN_3O_8$ requires C, 47.56; H, 5.32; N, 7.92 %.

(1H, m, 3'CH), 6.26 (1H, t, J = 6Hz, 1'CH), 6.9 (1H, d, J = 14Hz, CH=CHBr), 7.4 (1H, d, J = 14Hz, CH=CHBr), 7.92 (1H, s, 6-CH), 8.65 (6H, br.s, $D_2O$ exchangeable, 2 x $NH_3$), 11.6 (1H, s, $D_2O$ exchangeable, 3-NH).

Analysis

Found: C, 36.39; H, 3.96; N, 6.32 %. $C_{25}H_{33}BrF_6N_4O_{11}S_2$ requires C, 36.46; H, 4.04; N, 6.80 %.

Example 33

E-5-(2-Bromovinyl)-3',5'-di-L-valyl-2'-deoxyuridine bis trifluoroacetate

νmax (nujol) 1750, 1670-1740, 1620, 1600 $cm^{-1}$; $^1H$ nmr [$(CD_3)_2SO$] δ 0.95 (12H, m, 4 x $CH_3$), 2.0-2.5 (4H, m, 2'$CH_2$ + 2 x $CH(CH_3)_2$), 3.9 (2H, t, J = 6Hz, 2 x $CHNH_3$), 4.27 (1H, m, 4'CH), 4.4 (2H, m, 5'$CH_2$), 5.4 (1H, m, 3'CH), 6.27 (1H, t, J = 6Hz, 1'CH), 6.9 (1H, d, J = 14Hz, CH=CHBr), 7.35 (1H, d, J = 14Hz, CH=CHBr), 7.94 (1H, s, 6-CH), 8.61 (7H, br.s, $D_2O$ exchangeable, 2 x $NH_3$ + 3-NH).

Analysis

Found: C, 39.78; H, 4.16; N, 7.41 %. $C_{25}H_{33}BrF_6N_4O_{11}$ requires C, 39.54; H, 4.38; N, 7.38 %.

Example 34

E-5-(2-Bromovinyl)-3',5'-di-L-isoleucyl-2'-deoxyuridine bis trifluoroacetate

νmax (nujol) 1760, 1680-1740, 1600 $cm^{-1}$; $^1H$ nmr [$(CD_3)_2SO$] δ 0.9 (12H, m, 4 x $CH_3$), 1.35 (4H, m, 2 x $CH_2CH_3$), 1.86 (2H, m, 2 x $CHCH_3$) 2.4 (2H, m, 2'$CH_2$), 4.0 (2H, t, J = 6Hz, 2 x $CH\overset{+}{N}H_3$), 4.3 (1H, m, 4'CH), 4.45 (2H, m, 5'$CH_2$), 5.39 (1H, m, 3'CH), 6.26 (1H, t, J = 6Hz, 1'CH), 6.88 (1H, d, J = 14Hz, CH=CHBr), 7.36 (1H, d, J = 14Hz, CH=CHBr), 7.91 (1H, s, 6-CH), 8.6 (6H, br.s, $D_2O$ exchangeable, 2 x $NH_3$), 11.7 (1H, s, $D_2O$ exchangeable, 3-NH).

Analysis

Found: C, 40.89; H, 4.74; N, 7.06 %. $C_{27}H_{37}BrF_6N_4O_{11}$ requires C, 41.18; H, 4.74; N, 7.11 %.

BAD ORIGINAL

Example 35

E-5-(2-Bromovinyl)-3',5'-di-L-prolyl-2'-deoxyuridine bis trifluoroacetate

νmax (nujol) 1760, 1680-1740, 1600 $cm^{-1}$; $^{1}H$ nmr $[(CD_3)_2SO]$ δ 1.8-2.5 (10H, m, 4 x $CH_2$ + 2'$CH_2$), 3.26 (4H, t, J = 6Hz, 2 x $\underline{CH}\overset{+}{N}H_2$), 5.35 (1H, m, 3'CH), 6.2 (1H, t, J = 6Hz, 1'CH), 6.9 (1H, d, J = 14Hz, $\underline{CH}$=CHBr), 7.34 (1H, d, J = 14Hz, CH=$\underline{CH}$Br), 7.9 (1H, s, 6-CH), 9.62 (4H, br.s, $D_2O$ exchangeable, 2 x $\overset{+}{N}H_2$).

Analysis

Found: C, 39.47; H, 3.81; N, 7.33 %. $C_{25}H_{29}BrF_6N_4O_{11}$ requires C, 39.75; H, 3.87; N, 7.42 %.

Example 36

E-5-(2-Bromovinyl)-5'-L-prolyl-2'-deoxyuridine trifluoroacetate

νmax (nujol) 1760, 1670-1740, 1600 $cm^{-1}$; $^{1}H$ nmr $[(CD_3)_2SO]$ δ 1.91 (4H, m, 2 x $CH_2$), 2.24 (2H, m, 2'$CH_2$), 3.21 (2H, m, $CH_2$-$\overset{+}{N}H_2$), 3.93 (1H, m, 4'CH), 4.37 (3H, m, 5'$CH_2$ + 3'CH), 5.65 (1H, br.s, $D_2O$ exchangeable, OH), 6.17 (1H, t, J = 6Hz, 1'CH), 6.9 (1H, d, J = 14Hz, $\underline{CH}$=CHBr), 7.32 (1H, d, J = 14Hz, CH=$\underline{CH}$Br), 9.4 (5H, br.s, $D_2O$ exchangeable, 2 x $\overset{+}{N}H_2$ + 3-NH).

Analysis

Found: C, 39.77; H, 3.69; N, 7.63 %. $C_{18}H_{21}BrF_3N_3O_8$ requires C, 39.73; H, 3.89; N, 7.72 %.

Example 37

E-5-(2-Bromovinyl)-3'-L-prolyl-2'-deoxyuridine trifluoroacetate

νmax (nujol) 1750, 1670-1740, 1600 $cm^{-1}$; $^{1}H$ nmr $[(CD_3)_2SO]$ δ 1.95 (4H, m, 2 x $CH_2$), 2.3 (2H, m, 2'$CH_2$), 3.22 (2H, m, $\underline{CH_2}\overset{+}{N}H_2$), 3.65 (2H, m, 5'$CH_2$), 4.1 (1H, m, 4'CH), 4.15-4.8 (2H, m, $\underline{CH}\overset{+}{N}H_2$ + OH), 5.34 (1H, m, 3'CH),

Cont'd./...

BAD ORIGINAL

0080305

General method for the trifluoroacetic acid deprotection of E-5-(2-Bromovinyl)-2'-deoxyuridine-5'(3')- and 3',5'-di-(t-butoxycarbonylaminoacyl) esters (Examples 30-39)

A solution of E-5-(2-bromovinyl)-2'-deoxyuridine-5'(3')- or 3',5'-di-(t-butoxycarbonylaminoacyl) ester (0.5mmol) in trifluoroacetic acid (2mL) was stirred at 0$^{o}$ for 15 minutes. The solvent was removed under reduced pressure and the foamy residue triturated twice with dry ether to give the salts as white solids.

The following compounds were prepared by this method.

| Example No. | Yield % |
|---|---|
| Example 30<br>E-5-(2-Bromovinyl)-3',5'-di-D-leucyl-2'-deoxyuridine bis trifluoroacetate | 92 |
| Example 31<br>E-5-(2-Bromovinyl)-3',5'-di-D-phenylalanyl--2'-deoxyuridine bis trifluoroacetate | 87 |
| Example 32<br>E-5-(2-Bromovinyl)-3',5'-di-L-methionyl-2'-deoxyuridine bis trifluoroacetate | 94 |
| Example 33<br>E-5-(2-Bromovinyl)-3',5'-di-L-valyl-2'-deoxyuridine bis trifluoroacetate | 91 |
| Example 34<br>E-5-(2-Bromovinyl)-3',5'-di-L-isoleucyl-2'-deoxyuridine bis trifluoroacetate | 82 |
| Example 35<br>E-5-(2-Bromovinyl)-3',5'-di-L-prolyl-2'-deoxyuridine bis trifluoroacetate | 96 |
| Example 36<br>E-5-(2-Bromovinyl)-5'-L-prolyl-2'-deoxyuridine trifluoroacetate | 98 |
| Example 37<br>E-5-(2-Bromovinyl)-3'-L-prolyl-2'-deoxyuridine trifluoroacetate | 97 |
| Example 38<br>E-5-(2-Bromovinyl)-3',5'-di-L-alanyl-2'-deoxyuridine bis trifluoroacetate | 88 |
| Example 39<br>E-5-(2-Bromovinyl)-3',5'-di-L-glutamyl- | 93 |

BAD ORIGINAL

Example 30

E-5-(2-Bromovinyl)-3',5'-di-D-leucyl-2'-deoxyuridine bis trifluoroacetate

νmax (nujol) 1755, 1670-1740 (broad), 1600 $cm^{-1}$; $^1H$ nmr [$(CD_3)_2SO$] δ 0.9 (12H, m, 4 x $CH_3$), 1.65 (6H, m, 2 x $CH_2CH$), 2.45 (2H, m, 2'$CH_2$), 4.03 (1H, m, 2 x $\underline{CH}\overset{+}{N}H_3$), 4.2-4.5 (3H, m, 5'$CH_2$ + 4'CH), 5.36 (1H, m, 3'CH), 6.24 (1H, t, J = 6Hz, 1'CH), 6.9 (1H, d, J = 14Hz, <u>CH</u>=CHBr), 7.36 (1H, d, J = 14Hz, CH=<u>CH</u>Br), 7.93 (1H, s, 6-CH), 8.6 (7H, br.s, $D_2O$ exchangeable, 2 x $NH_3$ + 3-NH).

Analysis

Found: C, 41.43; H, 4.57; N, 7.18 %. $C_{27}H_{37}BrF_6N_4O_{11}$ requires C, 41.18; H, 4.74; N, 7.11 %.

Example 31

E-5-(2-Bromovinyl)-3',5'-di-D-phenylalanyl-2'-deoxyuridine bis trifluoroacetate

νmax (nujol) 1760, 1680-1740 (broad) $cm^{-1}$; $^1H$ nmr [$(CD_3)_2SO$] δ 2.15 (2H, m, 2'$CH_2$), 3.2 (4H, m, 2 x Ph$\underline{CH_2}$), 4.1-4.6 (5H, m, 4'CH + 5'$CH_2$ + 2 x $\underline{CH}CH_2$), 5.25 (1H, m, 3'CH), 6.14 (1H, t, J = 6Hz, 1'CH), 6.94 (1H, d, J = 14Hz, <u>CH</u>=CHBr), 7.35 (11H, m, aromatic + CH=<u>CH</u>Br), 7.94 (1H, s, 6-CH), 8.7 (7H, br.s, $D_2O$ exchangeable, 2 x $NH_3$ + 3-NH).

Analysis

Found: C, 46.11; H, 3.90; N, 6.48 %. $C_{33}H_{33}BrF_6N_4O_{11}$ requires C, 46.33; H, 3.89; N, 6.55 %.

Example 32

E-5-(2-Bromovinyl)-3',5'-di-L-methionyl-2'-deoxyuridine bis trifluoroacetate

νmax (nujol) 1760, 1650-1740, 1600 $cm^{-1}$; $^1H$ nmr [$(CD_3)_2SO$] δ 2.0 (3H, s, $CH_3S$), 2.07 (3H, s, $CH_3S$), 2.1 (4H, m, 2 x $\underline{CH_2}CH$), 2.55 (4H, m, 2'$CH_2$ + 2 x $CH_2S$), 4.17 (2H, m, 2 x $\underline{CH}\overset{+}{N}H_3$), 4.3-4.6 (3H, m, 5'$CH_2$ + 4'CH), 5.37

Cont'd./...

BAD ORIGINAL

6.2 (1H, t, J = 6Hz, 1'CH), 6.85 (1H, d, J = 14Hz, CH=CHBr), 7.27 (1H, d, J = 14Hz, CH=CHBr), 8.1 (1H, s, 6-CH), 9.5 (5H, br.s, $D_2O$ exchangeable, 2 x $\overset{+}{N}H_2$ + 3-NH).

Analysis

Found: C, 40.06; H, 3.44; N, 7.35 %. $C_{18}H_{21}BrF_3N_3O_8$ requires C, 39.72; H, 3.89; N, 7.72 %.

Example 38

E-5-(2-Bromovinyl)-3',5'-di-L-alanyl-2'-deoxyuridine bis trifluoroacetate

νmax (nujol) 1760, 1670-1730, 1600 $cm^{-1}$; $^1H$ nmr [$(CD_3)_2SO$] δ 1.46 (6H, d, J = 7.5Hz, 2 x $CH_3$), 2.48 (2H, m, 2'$CH_2$), 4.13 (2H, m, 2 x $CH\overset{+}{N}H_3$), 4.27 (1H, m, 4'CH), 4.4 (2H, m, 5'$CH_2$), 5.37 (1H, m, 3'CH), 6.2 (1H, t, J = 6Hz, 1'CH), 6.89 (1H, d, J = 14Hz, CH=CHBr), 7.33 (1H, d, J = 14Hz, CH=CHBr), 7.9 (1H, s, 6-CH), 8.57 (6H, br.s, $D_2O$ exchangeable, 2 x $\overset{+}{N}H_3$), 11.68 (H, br.s, $D_2O$ exchangeable, 3-NH).

Analysis

Found: C, 34.89; H, 3.40; N, 7.46 %. $C_{21}H_{35}BrF_6N_4O_{11}$ requires C, 35.56; H, 3.55; N, 7.90 %.

Example 39

E-5-(2-Bromovinyl)-3',5'-di-L-glutamyl-2'-deoxyuridine bis trifluoroacetate

νmax (nujol) 1760, 1660-1630 $cm^{-1}$; $^1H$ nmr [$(CD_3)_2SO$] δ 2.05 (4H, m, 2 x $CH_2CH$), 2.28 (4H, m, 2 x $CH_2\overset{O}{\overset{\|}{C}}NH_2$), 2.45 (2H, m, 2'$CH_2$), 4.15 (2H, m, 2 x $CH\overset{+}{N}H_3$), 4.35 (1H, m, 4'CH), 4.5 (2H, m, 5'$CH_2$), 5.39 (1H, m, 3'CH), 6.24 (1H, t, J = 6Hz, 1'CH), 6.94 (1H, d, J = 14Hz, CH=CHBr), 6.95 (1H, br.s, $D_2O$ exchangeable, $\overset{O}{\overset{\|}{C}}NH_2$), 7.34 (1H, d, J = 14Hz, CH=CHBr), 7.39 (1H, br.s, $D_2O$ exchangeable, $\overset{O}{\overset{\|}{C}}NH_2$), 7.87 (1H, s, 6-CH), 8.54 (6H, br.s, $D_2O$ exchangeable, 2 x $\overset{+}{N}H_3$), 11.76 (1H, br.s, $D_2O$ exchangeable, 3-NH).

Analysis

Found: C, 37.19; H, 4.00; N, 8.90 %. $C_{25}H_{31}BrF_6N_6O_{13}$ requires C, 36.73; H, 3.82; N, 10.27 %.

BAD ORIGINAL

Example 40

E-5-(2-Bromovinyl)-3',5'-di-L-lysyl-2'-deoxyuridine tetrakis trifluoroacetate

A solution of E-5-(2-bromovinyl)-3',5'-bis-($N_\alpha$,$N_\varepsilon$-di-t-butoxycarbonyl-L-lysyl)-2'-deoxyuridine (example 26) (600mg, 0.61mmol) in trifluoroacetic acid (6mL) was stirred at $0^o$ for 30 minutes. The solvent was removed under reduced pressure and the foamy residue triturated with dry ether to give a white solid which was dissolved in water (10mL) and freeze dried to give E-5-(2-bromovinyl)-3',5'-di-L-lysyl-2'-deoxyuridine tetrakis trifluoroacetate dihydrate as a white foam in 60% yield (380mg). νmax (nujol) 1760, 1600-1730 $cm^{-1}$; $^1H$ nmr [$(CD_3)_2SO$] δ 1.45 (8H, m, 2 x $H_3\overset{+}{N}CH_2(\underline{CH_2})_2$), 1.74 (4H, m, 2 x $\underline{CH_2}CH$), 2.7 (4H, m, 2 x $\underline{CH_2}\overset{+}{N}H_3$), 3.95 (2H, m, 2 x $\underline{CH}\overset{+}{N}H_3$), 4.28 (1H, m, 4'CH), 4.4 (2H, m, 5'$CH_2$), 5.33 (1H, m, 3'CH), 6.19 (1H, t, J = 6Hz, 1'CH), 6.84 (1H, d, J = 14Hz, $\underline{CH}$=CHBr), 7.3 (1H, d, J = 14Hz, CH=$\underline{CH}$Br), 7.83 (1H, s, 6-CH), 8.2 (12H, br.s, $D_2O$ exchangeable, 4 x $\overset{+}{N}H_3$), 11.65 (1H, br.s, $D_2O$ exchangeable, 3-NH),

Analysis

Found: C, 34.47; H, 4.18; N, 7.71 %. $C_{31}H_{41}BrF_{12}N_6O_{15}.2H_2O$ requires C, 34.41; H, 4.16; N, 7.77 %.

Example 41

E-5-(2-Bromovinyl)-3',5'-di-L-leucyl-2'-deoxyuridine bis trihydrogenpyrophosphate

A solution of E-5-(2-bromovinyl)-3',5'-di-(t-butoxycarbonyl-L-leucyl)-2'-deoxyuridine (150mg, 0.2mmol) in dry ether (25mL), was treated with a solution of pyrophosphoric acid (80mg, 0.45mmol) in dry tetrahydrofuran (5mL) and the solution stirred at room temperature for 1 hour. The solvent was removed under reduced pressure and the oily residue triturated twice with ether affording E-5-(2-bromovinyl)-3',5'-di-L-leucyl-2'-deoxyuridine bis trihydrogenphosphate (92mg, 51%) as a white solid.

$\nu_{max}$ (nujol) 1760, 1700, 1680 $cm^{-1}$; $^1H$ nmr [$(CD_3)_2SO$] $\delta$ 0.94 (12H, m, $4xCH_3$), 1.64 ($2x\underline{CH_2CH}(CH_3)_2$), 2.47 (2H, m, $2'CH_2$), 3.95 (2H, m, $2x\underline{CH}NH_3$), 4.15-4.6 (3H, m, $4'CH+5'CH_2$), 5.38 (1H, m, 3'CH), 6.21 (1H, m, 1'CH), 6.88 (1H, d, J=14Hz, $\underline{CH}$=CHBr), 7.35 (1H, d, J=14Hz, CH=$\underline{CH}$Br), 7.85 (1H, s, 6-CH), 7.95 (13H, m, $D_2O$ exchangeable, $2xNH_3.H_3P_2O_7$ + 3NH).

Analysis

Found: C, 31.21; H, 4.79; N, 5.61%. $C_{23}H_{43}BrN_4O_{21}P_4$ requires: C, 30.18; H, 4.73; N, 6.12%.

Example 42

E-5-(2-Bromovinyl)-3',5'-di-L-leucyl-2'-deoxyuridine bis formate

A solution of E-5-(2-bromovinyl)-3',5'-di-(t-butoxycarbonyl-L-leucyl)-2'-deoxyuridine (300mg, 0.4mmol) was dissolved in formic acid (8mL) and the solution stirred at room temperature for 3 hours. The solvent was removed under high vacuum with a bath temperature less than 30°C. The oily residue was repeatedly triturated with dry ether until a white solid formed and E-5-(2-bromovinyl)-3',5'-di-L-leucyl-2'-deoxyuridine bis formate (210mg, 82%) filtered off.

$\nu$max (nujol) 1760, 1700, 1680 $cm^{-1}$; $^1H$ nmr [$(CD_3)_2SO$] $\delta$ 0.89 (12H, m, 4x$CH_3$), 1.40 (4H, m, 2x$\underline{CH_2}$CH), 1.64 (2H, m, 2x$\underline{CH}(CH_3)_2$), 2.4 (2H, m, 2'$CH_2$), 3.43 (2H, t, J=6Hz, 2x$\underline{CH}NH_3$), 4.27 (3H, m, 5'$CH_2$+4'CH), 5.26 (1H, m, 3'CH), 5.85 (9H, m, $D_2O$ exchangeable, 2x$NH_3.HCO_2$+3NH), 6.16 (1H, t, J=7Hz, 1'CH), 6.88 (1H, d, J=14Hz, $\underline{CH}$=CHBr), 7.3 (1H, d, J=14Hz, CH=$\underline{CH}$Br), 7.83 (1H, s, 6-CH).

Analysis

Found: C, 45.98; H, 5.78; N, 8.07%. $C_{25}H_{39}BrN_4O_{11}$ requires: C, 46.09; H, 6.03; N, 8.59%.

## ANTIVIRAL ACTIVITY

### In Vitro

### Method

Vero (African Green Monkey Kidney) cells were grown to confluence in 6 well multidishes, each well being 3.5 cm in diameter. The cells were incubated with Herpes simplex type 1 virus (HFEM strain) and overlaid with 0.5 mL of 0.9% agarose (w/v) in maintenance medium containing the test compound at a range of concentrations from 3 µg/mL in half-log dilution steps. The virus infected cultures were then incubated at 37°C for 6 days before fixing in 4% formaldehyde solution and staining with carbolfuchsin. The dishes were then examined to find what concentration of test compound causing a 50% reduction in the number of virus plaques formed ($PDD_{50}$ value) and the minimum concentration of test compound which killed the cell monolayer, leaving a clear zone devoid of cells and virus plaques (MTD).

<u>Results</u>

| Example No | $PDD_{50}$ | | MTD (µg/mL) |
|---|---|---|---|
| | µg/mL | µM | |
| 1 | 1.10 | 1.33 | >100 |
| 2 | 0.16 | 0.28 | >100 |
| 3 | 0.25 | 0.43 | >100 |
| 4 | 1.95 | 3.01 | >100 |
| 5 | 0.48 | 0.98 | >100 |
| 6 | 0.54 | 1.10 | >100 |
| 7 | 0.14 | 0.16 | >100 |
| 8 | 0.45 | 0.76 | >100 |
| 9 | 0.19 | 0.32 | >100 |
| 10 | 0.19 | 0.24 | >100 |
| 12 | 0.86 | 1.13 | 100 |
| 13 | 0.94 | 1.87 | >100 |
| 14 | 0.36 | 0.53 | >100 |
| 15 | 0.80 | 0.90 | >100 |
| 16 | 0.02 | 0.025 | >100 |
| 17 | 0.49 | 0.76 | >100 |
| 18 | 0.28 | 0.37 | >100 |
| 19 | >100 | | >100 |
| 20 | >100 | | >100 |
| 21 | 1.71 | 2.15 | >100 |
| 22 | >100 | | >100 |
| 23 | >100 | | >100 |
| 24 | 0.62 | 0.92 | >100 |
| 25 | 0.47 | 0.60 | >100 |
| 26 | 90 | 91 | >100 |
| 27 | 3.51 | 4.83 | 100 |
| 28 | 2.12 | 4.00 | 100 |
| 29 | 0.64 | 1.21 | 100 |
| 30 | 0.16 | 0.20 | >100 |
| 31 | 0.55 | 0.64 | >100 |
| 32 | 0.26 | 0.32 | >100 |
| 33 | 0.24 | 0.32 | >100 |
| 34 | 0.40 | 0.51 | >100 |
| 35 | 0.22 | 0.29 | >100 |
| 36 | 0.55 | 1.01 | >100 |
| 37 | 0.68 | 1.25 | >100 |
| 38 | 0.26 | 0.37 | >100 |
| 39 | 0.42 | 0.51 | >100 |
| 40 | 0.19 | 0.18 | >100 |
| 41 | 0.55 | 0.60 | >100 |
| 42 | | | |

CLAIMS

1. A compound of formula (I):

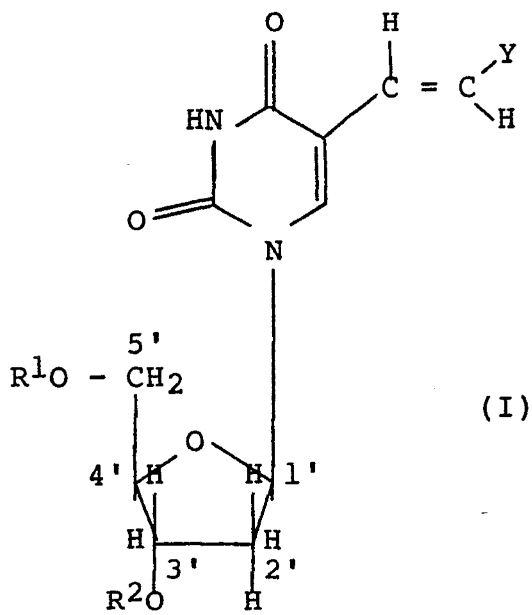

(I)

or a salt thereof,
in which Y is a halogen atom, and each of $R^1$ and $R^2$ is a hydrogen atom or an acylamino group of the formula (IA):

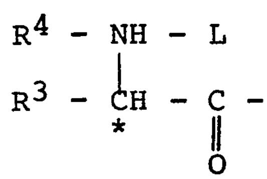

(IA)

which group is optionally N-alkylated or acylated, wherein $R^3$ is a hydrogen atom, or a $C_{1-6}$ alkyl, phenyl, optionally substituted $C_{1-6}$ phenylalkyl, $C_{1-6}$ hydroxyalkyl, $C_{1-6}$ aminoalkyl, optionally

N-substituted with $C_{1-6}$ alkoxycarbonyl, $C_{1-6}$ thioalkyl, $C_{1-6}$ alkylthioalkyl, $C_{1-6}$ carboxyalkyl or $C_{1-6}$ carboxamidoalkyl group, $R^4$ is a hydrogen atom or together with $R^3$ forms the remaining members of a heterocyclic ring, and L is a hydrogen atom or a protecting group, provided that $R^1$ and $R^2$ are not simultaneously hydrogen atoms.

2. A compound according to claim 1, in which Y is a bromine atom.

3. A compound according to claim 1 or claim 2, in which $R^3$ is a benzyl group optionally substituted by a halogen atom, or is a methyl, iso-propyl or iso-butyl group.

4. A compound according to claim 1 or claim 2, in which $R^3$ and $R^4$ complete a 5 to 7 membered saturated heterocyclic ring with no additional hetero-atom present therein.

5. The compound E-5-(2-bromovinyl)-3',5'-di-L-leucyl-2'-deoxyuridine or a pharmaceutically acceptable salt thereof.

6. A process for preparing a compound according to claim 1, which comprises treating a compound of formula (II):

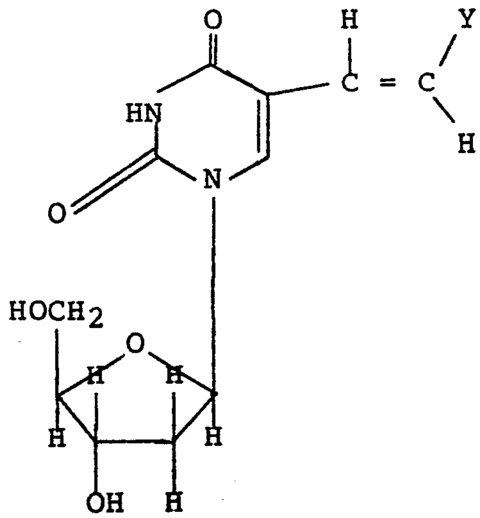

(II)

in which Y is as defined in formula (I), with a compound of formula (III):

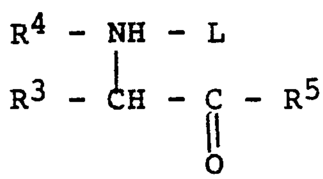

(III)

in which $R^3$ and $R^4$ are as defined in formula (IA), L is a protecting group, and $R^5$ is an active ester group, and optionally thereafter removing the protecting group L.

7. A process according to claim 6, in which $R^5$ is a N-hydroxy succinimide group or a p-nitrophenyl group.

8. A pharmaceutical composition comprising a compound according to any one of claims 1 to 5 together with a pharmaceutically acceptable carrier.

9. A compound according to any one of claims 1 to 5, for use in the treatment of viral infections.

European Patent Office

# EUROPEAN SEARCH REPORT

0080305
Application number

EP 82 30 6028

| DOCUMENTS CONSIDERED TO BE RELEVANT | | | |
| --- | --- | --- | --- |
| Category | Citation of document with indication, where appropriate, of relevant passages | Relevant to claim | CLASSIFICATION OF THE APPLICATION (Int. Cl. 3) |
| Y | GB-A-2 066 812 (FUJI KAGAKU KOGYO)<br>*Pages 1-4* | 1,8 | C 07 H 19/08<br>A 61 K 31/70 |
| A | GB-A-2 060 604 (UNIVERSITY OF BIRMINGHAM)<br>*Page 1* | 1,8 | |
| A | DE-A-3 010 399 (GAURI, K.)<br>*Pages 1,2* | 1,8 | |
| Y,P | EP-A-0 061 283 (BEECHAM)<br>*Pages 1-4* | 1,8 | |
| | | | TECHNICAL FIELDS SEARCHED (Int. Cl. 3) |
| | | | C 07 H 19/00<br>A 61 K 31/00 |

The present search report has been drawn up for all claims

| Place of search | Date of completion of the search | Examiner |
| --- | --- | --- |
| THE HAGUE | 21-02-1983 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document